(19) **Europäisches Patentamt European Patent Office Office européen des brevets**

(11) **EP 3 073 900 B1**

(12) **FASCICULE DE BREVET EUROPEEN**

(45) Date de publication et mention de la délivrance du brevet:
**23.08.2017 Bulletin 2017/34**

(21) Numéro de dépôt: **14803111.5**

(22) Date de dépôt: **25.11.2014**

(51) Int Cl.:
**A61B 5/11** (2006.01)    **A61B 5/00** (2006.01)
**G06T 7/269** (2017.01)    **H04N 7/18** (2006.01)
**A61B 7/00** (2006.01)    **H04N 5/14** (2006.01)

(86) Numéro de dépôt international:
**PCT/EP2014/075588**

(87) Numéro de publication internationale:
**WO 2015/078879 (04.06.2015 Gazette 2015/22)**

(54) **PROCÉDÉ DE CONSTRUCTION D'UN INDICE D'ACTIVITÉ, DISPOSITIF ET PROGRAMME D'ORDINATEUR CORRESPONDANT**

VERFAHREN ZUR KONSTRUKTION EINES AKTIVITÄTSINDEX, VORRICHTUNG UND COMPUTERPROGRAMM DAFÜR

METHOD FOR CONSTRUCTING AN ACTIVITY INDEX, DEVICE, AND COMPUTER PROGRAM THEREFORE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.11.2013 FR 1361719**

(43) Date de publication de la demande:
**05.10.2016 Bulletin 2016/40**

(73) Titulaires:
• **Université de Rennes I**
  **35000 Rennes (FR)**
• **Institut National de la Santé et de la Recherche Médicale**
  **75013 Paris 13 (FR)**
• **Centre Hospitalier Universitaire Pontchaillou**
  **35000 Rennes (FR)**

(72) Inventeurs:
• **POREE, Fabienne**
  **F-35000 Rennes (FR)**
• **SIMON, Antoine**
  **F-35000 Rennes (FR)**

• **PLADYS, Patrick**
  **F-35530 Noyal sur Vilaine (FR)**
• **CARRAULT, Guy**
  **F-35510 Cesson Sevigne (FR)**

(74) Mandataire: **Vidon Brevets & Stratégie**
  **16B, rue de Jouanet**
  **B.P. 90333**
  **Technopole Atalante**
  **35703 Rennes Cedex 7 (FR)**

(56) Documents cités:
**WO-A1-2013/106700    WO-A2-2004/032719**
**US-A- 5 689 241    US-A1- 2011 144 517**

• YA-TI PENG ET AL: "Multimodality Sensors for Sleep Quality Monitoring and Logging", DATA ENGINEERING WORKSHOPS, 2006. PROCEEDINGS. 22ND INTERNATIONAL CONFERENCE ON ATLANTA, GA, USA 03-07 APRIL 2006, PISCATAWAY, NJ, USA,IEEE, 3 avril 2006 (2006-04-03), pages x108-x108, XP010912001, DOI: 10.1109/ICDEW.2006.97 ISBN: 978-0-7695-2571-6

**Description**

**1 DOMAINE DE L'INVENTION**

L'invention se rapporte au domaine de la polysomnographie. Plus particulièrement, l'invention propose une nouvelle technique d'analyse du sommeil non invasive. Plus particulièrement encore, l'invention propose une technique d'obtention de variables physiologiques sans contact avec l'individu.

**2 SOLUTIONS DE L'ART ANTÉRIEUR**

Les techniques de polysomnographie sont nombreuses. Elles consistent à enregistrer, au cours du sommeil du patient, des variables physiologiques pour mettre en évidence des troubles liés au sommeil.

Parmi les variables physiologiques que l'on cherche à mesurer, on trouve par exemple le rythme respiratoire, le rythme cardiaque. On réalise également des mesures de l'activité du cerveau en utilisant des électroencéphalogrammes et des mesures de l'activité des muscles par un électromyogramme des muscles des bras ou des jambes...

Les mesures de ces différentes variables physiologiques nécessitent généralement d'appliquer, sur le corps du patient, de nombreux capteurs. Ces capteurs sont positionnés à différents endroits du corps en fonction de la variable physiologique à mesurer. Un premier problème de la polysomnographie réside justement dans ces nombreux capteurs : ils ne permettent pas au patient sur lequel on cherche à effectuer ces mesures d'atteindre un sommeil identique à celui qu'il atteint en temps normal (c'est-à-dire sans les capteurs). On comprend en effet qu'un patient sur lequel est posé un grand nombre d'électrodes, de sangles et de capteurs ne se trouve pas dans des conditions optimales pour trouver le sommeil.

Par ailleurs, ces types de capteurs sont relativement mal adaptés aux conditions de mesures dans lesquelles se trouvent les nouveau-nés ou jeunes enfants pour lesquels il est nécessaire d'effectuer une telle surveillance. En effet, pour ces patients, il est nécessaire de réaliser des enregistrements en continu, avec un nombre de capteurs limité, souvent dans un environnement de pénombre et à des stades comportementaux différents. Ainsi, les problématiques de captation de variables physiologiques sont plus complexes dans le cas du nouveau-né ou des prématurés que dans le cas de l'adulte.

La demande de brevet internationale publiée WO 2013/103700 A1, qui est considéré comme l'état de la technique le plus proche de la présente invention, décrit l'utilisation d'un flux audio/vidéo pour l'analyse du sommeil d'une personne.

Le brevet américain US 5689241 A décrit la détection de sommeil par la détermination de l'ouverture des yeux dans des images vidéo.

Or, à l'heure actuelle, les solutions pour résoudre ce problème sont peu nombreuses. Il existe donc un besoin de fournir une technique d'acquisition d'au moins certaines variables physiologiques par un système qui d'une part soit utilisable dans un cadre général d'obtention de variables physiologiques, par exemple chez l'adulte, et d'autre part soit adapté au cadre spécifique de captation de variables physiologiques chez le nouveau-né tout en évitant au maximum les appareillages et capteurs surnuméraires.

**3 RÉSUMÉ DE L'INVENTION**

L'invention ne pose pas ces problèmes liés aux techniques de l'art antérieur. En effet, l'invention concerne un procédé de calcul d'un indice d'activité d'un individu lors d'une phase de sommeil, mis en oeuvre par l'intermédiaire d'un dispositif de calcul d'indice.

Selon un mode de réalisation de la technique proposée, un tel procédé comprend :

- une étape d'obtention, à partir d'un flux audio /vidéo d'origine, d'au moins deux flux, chacun comprenant une pluralité d'échantillons horodatés ladite étape d'obtention comprenant une étape d'obtention de données représentatives d'un état des yeux, délivrant un flux comprenant des échantillons relatifs à l'ouverture des yeux dudit individu ;
- une étape de formatage desdits au moins deux flux, délivrant au moins deux flux formatés ;
- une étape de combinaison, desdits au moins deux flux formatés, délivrant un flux combiné ;
- une étape de calcul, à partir dudit flux combiné d'au moins un indice représentatif d'un niveau d'activité d'un individu.

Ainsi, la technique proposée permet de construire, de manière simple, un indice à partir d'un flux audio/vidéo déjà disponible. Cette technique ne nécessite pas de matériel complexe ou couteux et peut être implémentée en utilisant un flux audio/vidéo qui est déjà accessible et présent dans les systèmes de polysomnographie existants.

Selon un mode de réalisation particulier, ladite étape de combinaison met en oeuvre une fonction de fusion :

$$xc_j = ff(x_j) = \sum_{i=1}^{n} \propto_i x_{ij}$$

Dans laquelle :

- $n$ représente le nombre de flux ;
- $j$ représente le quantième de l'échantillon ;
- $x_{ij}$ est la valeur du flux normalisé $x_i$ au numéro d'échantillon $j$;
- $\alpha_i$ est une valeur de pondération du flux $x_i$.

Ainsi, cette fonction de fusion permet de combiner simplement des flux et de les présenter sous une forme normalisée.

Selon un mode de réalisation particulier, ladite valeur $\alpha_i$ de pondération du flux $x_i$ varie d'un quantième d'échantillon à un autre.

Ainsi, la fonction de fusion peut prendre en compte les différences entre les flux.

Selon une caractéristique particulière, ladite valeur $\alpha_i$ de pondération du flux $x_i$ est paramétrée en fonction d'au moins un flux $x_j, j \neq i$.

Ainsi, la fonction de fusion peut prendre en compte des informations contradictoires qui pourraient être issues des flux, comme par exemple une corrélation entre une fermeture des yeux et un volume sonore élevé.

Selon un mode de réalisation particulier, une étape d'obtention, à partir d'un flux audio /vidéo d'origine, d'au moins deux flux, comprend au moins une des étapes suivantes :

- une étape d'estimation de mouvement, délivrant un flux comprenant des échantillons relatifs à l'évaluation du mouvement entre des images successives dudit flux audio /vidéo d'origine ;
- une étape de collecte d'intensité sonore, délivrant un flux comprenant des échantillons relatifs à l'évaluation d'un volume sonore.

Dans un autre mode de réalisation, l'invention se rapporte également à un dispositif de calcul d'un indice d'activité d'un individu, mis en oeuvre par l'intermédiaire d'un dispositif de calcul d'indice.

Un tel dispositif comprend :

- des moyens d'obtention, à partir d'un flux audio /vidéo d'origine, d'au moins deux flux, chacun comprenant une pluralité d'échantillons horodatés ;
- des moyens de formatage desdits au moins deux flux, délivrant au moins deux flux formatés ;
- des moyens de combinaison, desdits au moins deux flux formatés, délivrant un flux combiné ;
- des moyens de calcul, à partir dudit flux combiné d'au moins un indice représentatif d'un niveau de repos d'un individu.

Selon les modes de réalisation, un tel dispositif peut être adjoint à un système de polysomnographie. Dans d'autres modes de réalisation, un tel dispositif peut se présenter sous une forme autonome, par exemple pour la surveillance des enfants en bas âge ou de personnes à mobilité réduite.

Selon une implémentation préférée, les différentes étapes des procédés selon l'invention sont mises en oeuvre par un ou plusieurs logiciels ou programmes d'ordinateur, comprenant des instructions logicielles destinées à être exécutées par un processeur de données d'un module relais selon l'invention et étant conçu pour commander l'exécution des différentes étapes des procédés.

En conséquence, l'invention vise aussi un programme, susceptible d'être exécuté par un ordinateur ou par un processeur de données, ce programme comportant des instructions pour commander l'exécution des étapes d'un procédé tel que mentionné ci-dessus.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un processeur de données, et comportant des instructions d'un programme tel que mentionné ci-dessus.

Le support d'informations peut être n'importe quelle entité ou dispositif capable de stocker le programme. Par exemple, le support peut comporter un moyen de stockage, tel qu'une ROM, par exemple un CD ROM ou une ROM de circuit microélectronique, ou encore un moyen d'enregistrement magnétique, par exemple une disquette (floppy disc) ou un disque dur.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

Selon un mode de réalisation, l'invention est mise en oeuvre au moyen de composants logiciels et/ou matériels. Dans cette optique, le terme "module" peut correspondre dans ce document aussi bien à un composant logiciel, qu'à un composant matériel ou à un ensemble de composants matériels et logiciels.

Un composant logiciel correspond à un ou plusieurs programmes d'ordinateur, un ou plusieurs sous-programmes d'un programme, ou de manière plus générale à tout élément d'un programme ou d'un logiciel apte à mettre en oeuvre une fonction ou un ensemble de fonctions, selon ce qui est décrit ci-dessous pour le module concerné. Un tel composant logiciel est exécuté par un processeur de données d'une entité physique (terminal, serveur, passerelle, routeur, etc.) et est susceptible d'accéder aux ressources matérielles de cette entité physique (mémoires, supports d'enregistrement, bus de communication, cartes électroniques d'entrées/sorties, interfaces utilisateur, etc.).

De la même manière, un composant matériel correspond à tout élément d'un ensemble matériel (ou hardware) apte à mettre en oeuvre une fonction ou un ensemble de fonctions, selon ce qui est décrit ci-dessous pour le module concerné. Il peut s'agir d'un composant matériel programmable ou avec processeur intégré pour l'exécution de logiciel, par exemple un circuit intégré, une carte à puce, une carte à mémoire, une carte électronique pour l'exécution d'un micrologiciel (firmware), etc.

Chaque composante du système précédemment décrit met bien entendu en oeuvre ses propres modules logiciels.

Dans un autre mode de réalisation, le procédé comprend les caractéristiques de la revendication 8 annexée ci-dessous.

Les différents modes de réalisation mentionnés ci-dessus sont combinables entre eux pour la mise en oeuvre de l'invention.

## 4 LISTE DES FIGURES

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :

- la figure 1 présente un synoptique de la technique proposée ;
- la figure 2 illustre la phase d'extraction de flux à partir du flux audio/vidéo d'origine ;
- la figure 3 illustre schématiquement un dispositif de mise en oeuvre de la technique proposée.

## 5 DESCRIPTION DÉTAILLÉE DE L'INVENTION

### 5.1 Rappel du principe de l'invention

Le principe général de la technique proposée réside dans l'extraction et le traitement combinatoire de données extraites d'une unique source audio/vidéo afin de générer un ou plusieurs indices différentiels d'activité d'un patient. Plus particulièrement, à la différence de ce qui est réalisé dans les techniques antérieures connues, l'utilisation de la source audio/vidéo est faite d'une part de manière prépondérante et d'autre part de manière automatique. En effet, il est certes fréquent de disposer d'un enregistrement vidéo lors de séances de polysomnographie. En revanche, cet enregistrement vidéo n'est pas utilisé de manière active pour déterminer l'état du patient, mais uniquement à titre de contrôle, réalisé par le médecin, de la séance de polysomnographie. Ainsi la technique proposée utilise un dispositif connu et présent, mais d'une manière complètement nouvelle par rapport à l'usage que l'on fait de ce dispositif. En règle générale, ce dispositif se présente sous la forme d'une caméra infrarouge ou d'une caméra en lumière visible associée à un enregistreur numérique. La méthode traditionnelle de scorage du sommeil (découpage du sommeil de l'individu en stades de sommeils) est longue et fastidieuse et génère de nombreuses erreurs (car l'avis du praticien est basé sur des critères fondés sur l'expérience et pas forcément sur des critères objectifs).

Au contraire l'invention permet d'une part de traiter le flux audio/vidéo de manière automatique et d'autre part avec des critères objectifs. Plus particulièrement, le principe général de la technique divulguée consiste à extraire de la source audio/vidéo, au moins deux flux de données différents et de combiner ces flux de données différents afin d'obtenir un indice.

Dans un mode de réalisation simple de l'invention, l'indice obtenu est un indice de calme. Cet indice permet de quantifier de manière grossière l'activité du patient ou son état d'agitation (qu'il soit éveillé ou endormi). Cet indice de calme est construit en obtenant à partir du flux audio/vidéo d'origine, deux flux (flux de mouvement et flux sonore). Ainsi, l'invention n'est nullement limitée à l'usage de trois composantes d'un flux audio/vidéo.

Dans d'autres modes de réalisation, basé sur un traitement particulier des données extraites, l'indice obtenu permet de qualifier, au moins partiellement, le stade du sommeil (sommeil profond, sommeil léger, somnolence, éveil tranquille, etc.) et permet de savoir si l'individu est éveillé ou endormi. Cette technique est particulièrement adaptée au nourrisson et au prématuré. Cet indice est appelé indice de repos et il est construit en obtenant à partir du flux audio/vidéo d'origine, trois flux (flux de mouvement, ouverture des yeux et flux sonore).

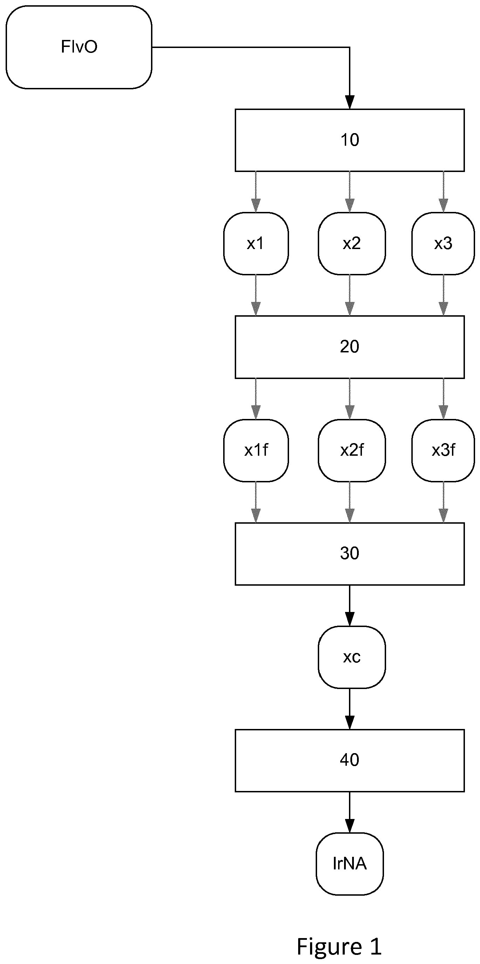

La méthode proposée, décrite en relation avec la figure 1, comprend quatre phases : une phase d'obtention de flux de données ; une phase de formatage des flux; une phase de combinaison, et une phase de calcul d'un indice.

Durant une phase d'obtention (10) de flux de données, les différents flux de données (x1, x2, x3) sont extraits à partir de la source audio/vidéo (Flvo), à l'aide de méthodes d'obtention spécifiques aux informations recherchées, dont certaines sont explicitées par la suite; ces flux de données sont, bien entendu, marqués temporellement ;

Puis, pour chacun des flux de données obtenus on met en oeuvre une phase de formatage (20) comprenant :

- une étape de détection d'enveloppe pour le flux audio, lorsqu'il en existe un, afin de le transformer en un signal positif ;
- une étape de lissage des données obtenue par le calcul d'une moyenne glissante sur une fenêtre de durée choisie préalablement ;
- une étape de rééchantillonnage puisque les différents flux possèdent des fréquences d'échantillonnage différentes ; l a fréquence d'échantillonnage finale peut être, par exemple, égale à la plus petite des différentes fréquences (en respectant les conditions d'échantillonnage) ;
- une étape de normalisation.

À l'issue de ces étapes, chacun des flux formatés *(x1f, x2f, x3f)* est présenté sous une échelle temporelle et une échelle de valeur communes aux autres flux. Bien entendu, en fonction de la nature des flux, certaines des étapes précédemment mentionnées peuvent être omises (c'est par exemple le cas d'un flux binaire dont les valeurs sont nécessairement comprises entre 0 et 1, et qui ne nécessite donc pas, a priori, de normalisation).

La méthode comprend ensuite une phase de combinaison (30), d'au moins deux des flux formatés *(x1f, x2f, x3f).*

Cette phase de combinaison étant mise en oeuvre par une fonction, dite fonction de fusion *(ff).* Cette fonction de fusion, dans sa forme la plus simple, peut être écrite de la façon suivante :

$$xc_j = ff(x_j) = \sum_{i=1}^{n} \propto_i x_{ij}$$

Dans laquelle :

- $xc_j$ est la valeur du flux résultant à l'échantillon $j$
- $n$ représente le nombre de flux ;
- $j$ représente le quantième de l'échantillon ;
- $x_{ij}$ est la valeur du flux normalisé $x_i$ au numéro d'échantillon $j$ ;
- $\alpha_i$ est une valeur de pondération du flux normalisé $x_i$ ;

Cette fonction de fusion délivre un flux combiné *(xc)*, échantillonné à la même échelle que les flux d'origine. La valeur de pondération $\alpha_i$ de chaque flux est comprise entre 0 et 1 et permet d'attribuer, à chacun des flux, une importance spécifique dans la fusion. Dans un mode de réalisation simple, cette valeur de pondération est égale à 1. Dans un mode de réalisation complexe, tel que présenté par la suite par exemple, cette pondération peut être évolutive (c'est-à-dire que la pondération évolue en fonction du flux auquel la pondération est affectée ou d'un autre flux, lié au flux courant et en fonction du temps). La valeur de pondération peut également être linéaire ou non linéaire, en fonction des modes de réalisation.

Ce flux combiné (xc) est représentatif de l'état de sommeil dans lequel se trouve l'individu.

Ainsi, par exemple, dans le cas de trois flux d'origine *(x₁, x₂, x₃),* le flux combiné *(xc)* est une suite d'échantillons dont chaque valeur est comprise entre « 0 » et « 3 ». Ainsi, par exemple, à intervalle régulier, dans l'échelle de valeur entière située entre « 0 » et « 3 » (i.e. « 0 », « 1 », « 2 » et « 3 »), on peut associer à chacune de ces valeurs entières un ou plusieurs états réel de l'individu (par exemple « 0 » correspond à un état de sommeil (profond ou léger) tandis que « 3 » correspond à un état d'éveil (calme ou agité)).

Dans un mode de réalisation complémentaire, un exemple de fonction de fusion pourrait être la suivante :

$$ff\left(x_j\right) = \frac{\sum_{i=1}^{n} \propto_i x_{ij}}{n}$$

Dans ce mode de réalisation, chaque valeur de ce flux combiné *(xc)* est normalisée, puisque moyennée. Les pondérations $\alpha_i$ peuvent être choisis par exemple en tenant compte des spécificités-sensibilités sur chacun des flux, ou bien encore en tenant compte de connaissance *a priori* sur le flux. Ces pondérations peuvent être des simples poids ou bien encore des coefficients évoluant avec le temps.

D'autres modes de réalisations sont possibles, on peut citer :

- les algorithmes de type réseau de neurones, où le réseau de neurones est appliqué à un vecteur de flux : après une phase d'apprentissage permettant d'ajuster les paramètres internes du réseau de neurones, chaque nouveau vecteur traité par le réseau de neurones se verra affecter en sortie une classe d'appartenance parmi les deux (ou plus) classes possibles.
- une pondération par filtre de Volterra directe : il s'agit d'un filtrage non-linéaire qui pondère les différents flux en fonction de noyaux $h_i$

$$ff(x_j) = \sum_{i=1}^{n} h_i x_{ij} + \ldots + \ldots + \sum_{i=1}^{n} \ldots \sum_{k=1}^{n} h_{i..k} x_{ij} \ldots x_{kj}$$

où le noyau $h_1$ représente la partie linéaire et les noyaux $h_{i..k}$ la partie non-linéaire.
- la réalisation d'un arbre de décision. Celui-ci correspond à une succession de tests, consistant à comparer les valeurs d'un flux à un ou plusieurs seuils. On aboutit alors à un flux de valeurs discrètes.

Comme l'objectif de la technique proposée est d'obtenir un résultat synthétique de l'activité du patient, les données constituant le flux sont ensuite traitées (40) pour aboutir à un indice représentatif du niveau d'activité *(IrNA)*.

Dans un premier mode de réalisation, on calcule des grandeurs statistiques, comme la moyenne, la variance du flux issu de la fusion.

Dans un second mode de réalisation le flux combiné *(xc)* est comparé à un ou plusieurs seuils ($s_{x1}$, $s_{x2}$, $s_{x3}$). Sur une période donnée (c'est-à-dire sur un nombre d'échantillons donnés parmi le nombre d'échantillons composants le flux) on compare le nombre de valeurs d'échantillons au-dessous du seuil par rapport au nombre de valeurs au-dessus du seuil afin d'obtenir un indice (par exemple un indice de repos, un indice de veille, un indice de sommeil, etc.). La valeur des seuils ($s_{x1}$, $s_{x2}$, $s_{x3}$) peut être fixée arbitrairement ou obtenue à partir d'une base d'apprentissage (par exemple une heure d'enregistrement) préalablement choisie, en utilisant des caractéristiques opérationnelles de réception (courbes COR).

Les différents indices peuvent être calculés sur des fenêtres de taille variable, par exemple 10 secondes ; on obtient alors un indice variant en fonction du temps. Ils peuvent aussi être calculés sur le temps total (une valeur globale sur tout l'enregistrement). De tels indices sont très utiles pour le corps médical.

5.2 Description d'un mode de réalisation

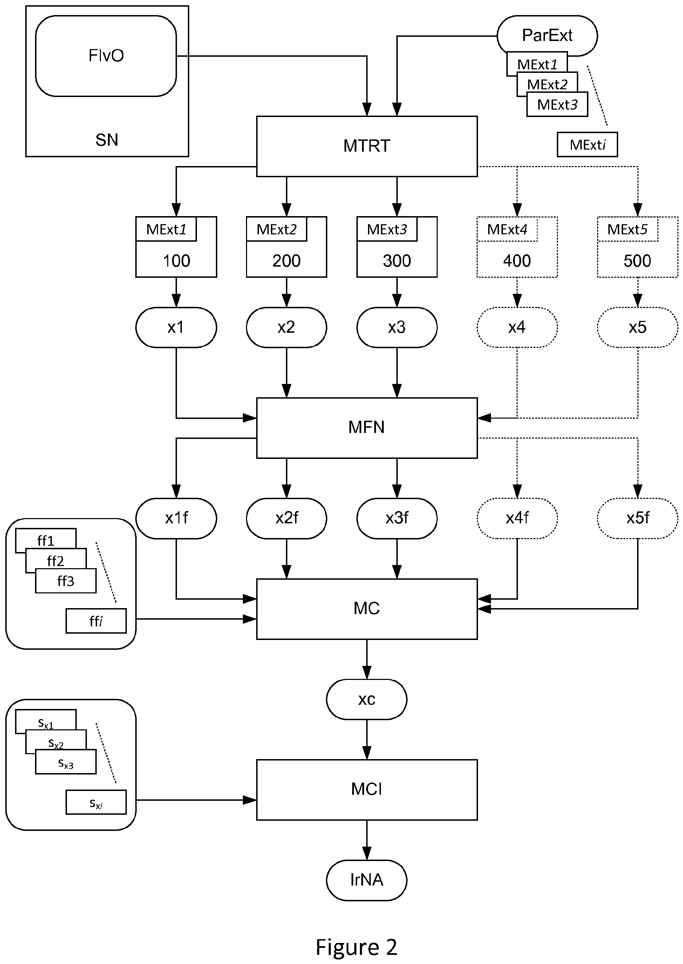

On présente, en relation avec la figure 2, la mise en oeuvre de la technique proposée à un flux audio/vidéo dans lequel on extrait trois flux de données différents : une estimation de mouvement, une extraction de bande sonore, une estimation de l'état des yeux.

Dans ce mode de réalisation, un flux audio/vidéo d'origine (*FlvO*) est enregistré sur un support numérique SN (de type CD, DVD, Disque Dur, etc). Un module de traitement (MTRT) reçoit en entrée d'une part le flux audio/vidéo d'origine (*FlvO*) et des paramètres d'extraction (ParExt). Les paramètres d'extraction définissent d'une part le nombre de flux à extraire et d'autre part, pour chacun des flux à extraire, une méthode d'extraction (MExt*i*), i correspondant à l'identifiant du flux.

Par exemple, en considérant que le premier flux (x1) se rapporte à l'estimation de mouvement, la méthode d'extraction peut être une différence inter-image ou le *flot optique,* c'est à dire les champs des vitesses mesurées à partir des variations de la luminance des différentes images. Cette méthode est intéressante dans le cas présent car pour des images peu bruitées, ne présentant pas de variations brutales de l'intensité et composées d'objets n'occasionnant pas de réflexions spéculaires prépondérantes, le "flot optique" est "globalement" assimilable à un mouvement projeté. D'autres méthodes d'estimation de mouvement peuvent également être mises en oeuvre, en fonction de paramètres propres au flux audio/vidéo d'origine (*FlvO*). À la différence des techniques existantes, cependant, l'objet de la mise en oeuvre d'une méthode d'estimation de mouvement n'est pas d'enregistrer des déplacements, mais d'estimer une quantité de déplacement. Cette estimation est donc bien plus simple à réaliser.

Ainsi, la technique proposée comprend une étape d'estimation de mouvement (100), délivrant un premier flux *(x1)* comprenant des échantillons relatifs à l'évaluation du mouvement entre images. Dans ce mode de réalisation, on obtient donc un flux *(x1)* comprenant un certain nombre d'échantillons (par exemple un toutes les secondes), chaque échantillon étant horodaté et ayant une valeur comprise entre 0 et 100, 0 signifiant qu'aucun mouvement n'est perçu alors que 100 signifie qu'une grande quantité de mouvement est perçue.

Le deuxième flux (x2), selon la méthode proposée est issue de la composante audio du flux audio/vidéo d'origine *(FlvO)*. Dans ce mode de réalisation, la méthode d'extraction consiste à mesurer (200) l'intensité de la piste audio au cours du temps. La méthode d'extraction (MExt2) est donc une méthode de mesure d'intensité. Cette mesure est soit absolue (c'est-à-dire qu'elle se base sur le contenu de la bande audio, sans référence), soit relative (c'est-à-dire qu'elle se base sur une échelle préalablement définie). Ce paramètre relatif à l'échelle a une importance dans le cas où des bruits ambiants sont présents et qu'il peut être nécessaire de ne pas les prendre en compte. Ainsi, dans le cas d'une mesure absolue, le niveau correspondant à une absence de bruit perçu en provenance du patient peut ne pas être immédiatement identifiable en cas de bruit ambiant. Il peut donc être nécessaire de mettre en oeuvre un traitement complémentaire consistant à éliminer (lorsque c'est possible) les bruits parasites et estimer la puissance du son ainsi que ses composantes fréquentielles pour différencier différents type de sons (cri, ronflement, pleurs, ...). Dans le cas d'une mesure d'intensité relative, on peut considérer que le niveau sonore à mesurer (le niveau sonore provenant du patient) dépasse forcément un niveau sonore de base.

Ainsi, la technique proposée comprend une étape d'enregistrement d'intensité sonore (200), délivrant un deuxième flux *(x2)* comprenant des échantillons relatifs à l'évaluation d'un volume sonore.

Le troisième flux *(x3)*, selon la méthode proposée est issu de la composante vidéo du flux audio/vidéo d'origine *(FlvO)*. Dans ce mode de réalisation, l'étape d'extraction (300) consiste à identifier une ouverture ou une fermeture des yeux. La méthode d'extraction (MExt3) est donc une méthode de détection d'ouverture ou de fermeture des yeux.

Pour ce faire, au moins trois méthodes d'extraction (MExt3) différentes peuvent être employées. La première méthode consiste à faire mesurer cette information par un praticien ou un manipulateur : tout au long du visionnage de la séquence, le praticien mesure cette ouverture, au besoin en utilisant une interface homme-machine particulière d'un programme informatique, qui autorise la saisie de l'état d'ouverture des yeux. L'utilisation de cette première méthode délivre un flux comprenant des échantillons binaires (indiquant si les yeux sont ouverts ou si les yeux sont fermés).

Une deuxième méthode, automatisée, consiste à tirer parti d'une propriété intéressante du fond de l'oeil soumis à une lumière infrarouge ou visible. À titre d'exemple ; une caractéristique des mesures de polysomnographie est qu'elles sont faites dans la pénombre. Ainsi, l'utilisation d'une caméra infrarouge est souvent nécessaire pour visualiser le déroulement de la mesure. Dès lors, le flux audio/vidéo d'origine *(FlvO)* comprend une composante vidéo infrarouge.

Or, une propriété de la captation d'image infrarouge est qu'elle rend le fond de l'oeil noir, c'est-à-dire parfaitement détachable du reste de l'image. Ainsi, il est possible, à l'aide d'un module de reconnaissance paramétré en conséquence, de distinguer la présence de noir dans la vidéo infrarouge. L'algorithme utilisé est un algorithme similaire à celui utilisé pour reconnaître et éliminer les yeux rouges dans une photographie avec flash. Cependant, l'algorithme proposé est plus simple car il ne nécessite pas de remplacer une portion d'image par une autre. Globalement, l'algorithme mis en oeuvre par ce module comprend d'une part une phase de seuillage dans laquelle ne sont conservées que les portions de l'image dont la valeur de noir est supérieure à un seuil déterminé (par exemple 80% ou 90%). Puis, à partir de cette image « seuillée », une recherche de zones noires est effectuée. L'objectif de cette recherche étant de déterminer la présence d'au moins deux zones, de forme sensiblement identique, et dont l'espacement de dépasse pas une valeur prédéterminée.

Lorsqu'au moins deux zones de ce type sont identifiées, l'algorithme se poursuit par une phase de calcul, à partir de ces zones, d'au moins un cercle dans lequel ces zones sont inscrites. Pour ce faire, pour au moins une des zones, à partir de la périphérie de celle-ci, un arc de cercle est tracé. Sur la base de la courbure de cet arc de cercle, une corde et une flèche sont obtenues. Puis le théorème de Pythagore est appliqué pour trouver le rayon du cercle correspondant. Le cercle obtenu est positionné sur chacune des zones afin d'éliminer les zones dont la surface est supérieure (d'un facteur prédéterminé) à la surface du cercle. Ceci permet d'éliminer les « faux positifs ». Cette phase de recherche est effectuée de manière itérative jusqu'à soit l'obtention de deux zones de forme sensiblement identique et dont la forme s'inscrit dans un cercle commun. Lorsqu'à l'issue de cette phase, aucune zone n'est identifiée, on considère que les yeux sont fermés.

Lorsque deux zones correspondant aux critères préalablement indiqués ont été identifiées, la dernière phase consiste à calculer l'aire de la zone noire par rapport à l'aire du cercle qui la compose pour obtenir un ratio d'ouverture de l'oeil (ratio compris entre 0 et 1).

Une troisième méthode consiste à combiner suivi et détection des yeux. Un processus de détection des yeux ouverts est d'abord mis en oeuvre. Il repose sur le fait que la pupille correspond à une région sombre entourée de pixels d'intensités claires. Cette caractéristique est accentuée lors d'une acquisition réalisée en infra-rouge. Le détecteur repose donc sur un seuillage permettant de sélectionner les pixels d'intensité faible, éventuellement une étape de morphologie mathématique pour supprimer les petites zones puis sur une sélection des régions de tailles comprises dans un intervalle prédéterminé (par un seuillage sur le nombre de pixels connexes). La valeur du périmètre ou de l'aire de la région fournit alors une information sur le degré d'ouverture de l'oeil.

Cette étape de détection est combinée à un processus de suivi. A partir d'une initialisation manuelle de la position des deux yeux, une région d'intérêt rectangulaire est déterminée autour de chaque oeil. Les étapes suivantes sont appliquées indépendamment pour chaque oeil. A chaque nouvelle image, la nouvelle position de l'oeil est recherchée autour de la position précédente. Pour ce faire, une fenêtre est déplacée dans la nouvelle image autour de la position précédente (suivant un critère de distance maximale). A chacune des positions de la fenêtre :

- le processus de détection des yeux ouverts est appliqué ;
- une métrique (somme des différences au carré entre les intensités de l'ensemble des pixels des fenêtres) est calculée entre la fenêtre initiale et la fenêtre testée.

Un règle de décision est mise en oeuvre pour déterminer la nouvelle position de l'oeil :

- si le processus de détection des yeux ouverts est positif en une position et une seule, cette position est sélectionnée et l'oeil est considéré comme étant ouvert ;
- si le processus de détection des yeux ouverts est positif en plusieurs positions et que l'état précédent de l'oeil était « ouvert », la position correspondant à la métrique la plus faible est sélectionnée et l'oeil est considéré comme étant ouvert ;
- si le processus de détection des yeux ouverts est positif en plusieurs positions et que l'état précédent de l'oeil était « fermé », la position la plus proche de la position précédente est sélectionnée et l'oeil est considéré comme étant ouvert ;
- si le processus de détection des yeux ouverts est négatif et que la métrique la plus faible est inférieure à un seuil de détection, la position correspondant à la métrique la plus faible est sélectionnée et l'oeil est considéré comme étant fermé ;
- si le processus de détection des yeux ouverts est négatif et que la métrique la plus faible est supérieur à un seuil de détection, le suivi de l'oeil est perdu et une nouvelle initialisation est demandée à l'utilisateur.

Cette opération automatique est répétée de manière régulière sur le flux audio/vidéo d'origine afin de délivrer un flux *(x3)* comprenant des échantillons horodatés dont la valeur est comprise entre 0 et 1.

Au besoin, après cette phase automatique, le flux obtenu peut être soumis, dans une dernière phase, à l'appréciation d'un opérateur afin de vérifier ou approuver la qualité du résultat obtenu.

À l'issue de ces différentes étapes de traitement, on dispose donc de plusieurs flux *(x1, x2, x3)* pouvant être traités selon les modalités présentées préalablement. Dans un mode de réalisation complémentaire, un quatrième flux *(x4)* est extrait du flux audio/vidéo. Il s'agit d'un flux relatif à la fréquence cardiaque du patient. En effet le rythme cardiaque peut aussi être estimé à partir d'un enregistrement vidéo acquis à l'aide d'une caméra classique ou thermique. Un cinquième flux *(x5)*, relatif à la respiration du patient peut aussi être extrait (par exemple par caméra thermique). Un tel flux de données, échantillonné et normalisé, permet de définir plus finement encore des paliers de sommeil ou des niveaux d'activité.

La méthode se poursuit par la mise en oeuvre de la normalisation, délivrant les flux « normés ». Cette normalisation est mise en oeuvre par un module de formatage et de normalisation (MFN). La combinaison des flux est mise en oeuvre par l'intermédiaire d'un module de combinaison (MC), qui utilise une ou plusieurs fonctions de fusion (ffi) adaptées aux flux à traiter. Puis le flux combiné (xc) est utilisé par un module de calcul d'indice (MCI) pour délivrer l'indice (IrNA) en fonction d'un ou plusieurs seuils ($s_{x1}$, $s_{x2}$, $s_{x3}$).

### 5.3 Formatage des flux de données

Dans ce mode de réalisation de la technique proposé, une caméra du commerce est utilisée pour effectuer la captation de la source audio/video (Flvo). Il en résulte une mise en oeuvre d'un formatage des flux ($x_x$) qui dépend de cette source d'origine. Le formatage présenté ici tient compte de cette captation d'origine. Une autre caméra ou un autre type de caméra peut évidemment fournir une source audio/video (Flvo) différente ce qui aura pour conséquence un échantillonnage d'origine différent et donc un formatage différent.

### 5.3.1 Signaux après la phase d'obtention

Après la phase d'obtention les différents flux ont des fréquences d'échantillonnages différentes :

- Le mouvement a été extrait depuis la vidéo et a donc la même fréquence d'échantillonnage que la vidéo 25 frames/sec : 25 Hz (1692*25=42300 échantillons).
- Le son extrait de la vidéo a une fréquence d'échantillonnage de 11,5 kHz, imposée par le fichier (1692*11500= 19458000 échantillons).
- Les yeux ont été scorés à chaque seconde : 1 Hz (1692 échantillons).

### 5.3.2 Cas particulier du son

Le signal représentant le son comprend deux spécificités :

a) il a une fréquence d'échantillonnage très élevée, qui n'est pas nécessairement utile, et qui fournit des signaux de grande taille. Un sous-échantillonnage est donc effectué.
b) contrairement aux deux autres flux, il comprend des valeurs positives et négatives. Une détection d'enveloppe (Transformée de Hilbert), est donc appliquée pour un signal positif.

### 5.3.3 Lissage

Dans le but d'obtenir des courbes plus « lisses », une moyenne glissante sur une fenêtre de taille donnée est calculée. Autrement dit, chaque point du signal d'origine est remplacé par la moyenne des points situés dans l'intervalle centré sur la fenêtre. À ce niveau du traitement les signaux n'ont toujours pas la même fréquence d'échantillonnage, la moyenne est donc calculée sur un nombre de points différent selon les signaux.

### 5.3.4 Rééchantillonnage

Pour pouvoir combiner les flux, il est intéressant avoir la même fréquence d'échantillonnage f_ech. Pour uniformiser les fréquences d'échantillonnage, il y a plusieurs possibilités. Il est ainsi possible de tout échantillonner à la plus petite des trois fréquences d'échantillonnage.
Il est aussi possible d'échantillonner les trois flux à une fréquence f_ech inférieure à fmin. Dans ce cas, les trois signaux sont sous-échantillonnés.
Enfin, il est aussi possible d'échantillonner les flux à une fréquence f_ech supérieure à fmin. il faut dans ce cas interpoler les signaux de fréquence d'échantillonnage inférieure à f_ech.

### 5.3.5 Normalisation

La dernière étape du formatage des données consiste à normaliser les trois flux entre 0 et 1. Il faut souligner que cette étape de normalisation entre 0 et 1 n'a du sens que si on évite le cas particulier où un flux serait proche de 0 (ou de 1) pendant toute la durée de l'enregistrement (aucun mouvement ou aucun son, ou les yeux toujours fermés ou ouverts).

### 5.4 Couplage avec d'autres flux

Dans un mode de réalisation complémentaire, les flux extraits du flux audio vidéo sont couplés avec d'autres flux *(xn)*, provenant d'un ou plusieurs autres dispositifs de mesure. L'acquisition d'un flux relatif à la fréquence cardiaque ou d'un flux relatif à la respiration est possible.
Pour le rythme cardiaque, outre les dispositifs classiques d'acquisition utilisant des électrodes, un oxymètre colorimétrique fournissant une mesure des pulsations cardiaques peut être utilisé.
Pour la respiration, il est possible d'utiliser les sangles placées autour de l'abdomen et du thorax qui enregistrent les mouvements respiratoires, ou un capteur naso-buccal qui enregistre le flux aérien ou encore un dispositif d'impédancemétrie utilisant des électrodes thoraciques.
Un tel flux de données, échantillonné et normalisé, permet de définir plus finement encore des paliers de sommeil ou des niveaux d'activité.
Dans un autre mode de réalisation, plusieurs caméras peuvent fournir plusieurs flux audio/vidéo. Par exemple deux caméras peuvent être positionnées à la place d'une seule caméra. Les flux qui sont extraits *(x1, x2, x3)* sont de même nature. On a par exemple deux flux pour le mouvement *(x1, x1')*, deux flux pour les yeux *(x3, x3')* et deux flux pour l'audio *(x2, x2')*. L'avantage de cette configuration est qu'avant de réaliser la fusion, chaque flux d'une paire de flux peut être corrélé avec l'autre flux de la paire, afin de fournir un flux résultant (par exemple x1") de meilleure qualité.

5.5 Autres caractéristiques optionnelles et avantages

Dans au moins un mode de réalisation de l'invention, un paramètre physiologique est adjoint à la phase combinatoire. Plus particulièrement, dans au moins un mode de réalisation, le paramètre physiologique adjoint est un paramètre comportemental de compensation. Ce paramètre est utilisé pour compenser ou inverser ou limiter la prise en compte d'un des flux de données en fonction d'un ou plusieurs autres flux de données. Ce paramètre physiologique détermine la valeur du coefficient $\alpha_i$ de pondération du flux normalisé $x_i$ auquel le paramètre est associé.

Par exemple, dans le cas d'un nouveau-né, il est fréquent que celui-ci ferme les yeux lors de pleurs. Dès lors, il est nécessaire de ne pas considérer que l'individu est endormi lorsque ses yeux sont fermés et qu'un niveau sonore relatif aux cris perçus excède un seuil prédéterminé.

Ainsi, dans ce cas, le paramètre comportemental de compensation comprend deux composantes : une composante définissant la relation entre l'état des yeux et les cris perçus dans le flux audio et une composante définissant une valeur plafonnée associée aux cris perçus dans le flux audio, valeur plafonnée qui, lorsqu'elle est dépassée par l'intensité du volume sonore des cris du flux audio déclenche l'annulation de la prise en compte de l'état associé à la fermeture des yeux (si le nouveau-né a les yeux fermés, il est alors considéré comme étant éveillé).

À titre d'exemple, le paramétrage du dispositif de mise en oeuvre de la présente technique peut être réalisé par une création, au sein d'un fichier de paramétrage, d'une structure de données, par exemple de type XML, dans laquelle le paramètre comportemental de compensation peut prendre une forme telle que la suivante :

```
<ParCC>
        <Rel>
                <flx1>f-audio</flx1>
                <flx2>f-eyes</flx2>
        </Rel>
        <Val1>
                <flx>f-audio</flx><val>0.9</val><cmp>upper</cmp>
                <state><flx>f-eyes</flx><pond>1</pond></state>
        </Val1>
</ParCC>
```

Concrètement, l'exemple précédent indique que le paramètre s'applique au flux audio et au flux d'ouverture des yeux. La valeur du paramètre indique que si le flux audio a une valeur normalisée supérieure à « 0,9 », alors la pondération du flux de la valeur des yeux est fixée à 1 (c'est-à-dire que les yeux sont considérés comme ouverts, même s'ils sont fermés).

Bien entendu, toute autre forme de paramétrage peut être définie. Il ne s'agit ici que de donner un exemple de la manière dont le dispositif peut être paramétré afin de fournir des indices qui soient représentatifs de la réalité, tout en étant réalisé de manière automatique.

5.6 Dispositif de mise en oeuvre

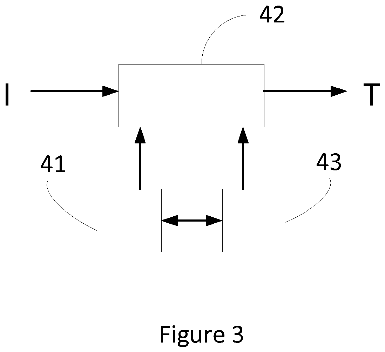

On présente, en relation avec la figure 3, une architecture simplifiée d'un dispositif apte à mettre en oeuvre la technique décrite. Un tel dispositif comprend une mémoire 41, une unité de traitement 42 équipée par exemple d'un microprocesseur, et pilotée par le programme d'ordinateur 43, mettant en oeuvre au moins une partie du procédé tel que décrit. Dans au moins un mode de réalisation, la technique décrite est mise en oeuvre sous la forme d'une application logicielle. Dans un autre mode de réalisation, la technique décrite est mise en oeuvre sous une forme purement matérielle, à l'aide de processeurs et d'interface spécialement créés à cet effet. Un tel dispositif comprend :

- des moyens d'obtention, à partir d'un flux audio /vidéo d'origine (FlvO), d'au moins deux flux parmi les trois (x1, x2, x3, ..., xn), chacun comprenant une pluralité d'échantillons horodatés ;
- des moyens de formatage desdits au moins deux flux (x1, x2, x3), délivrant au moins des flux formatés ;
- des moyens de combinaison, desdits au moins deux flux formatés, délivrant un flux combiné (xc) ;
- des moyens de calcul, à partir dudit flux combiné (xc) d'au moins un indice représentatif d'un niveau de repos d'un individu.

Ces moyens sont pilotés par le microprocesseur, à l'aide du programme chargé dans la mémoire du dispositif. En fonction des modes de réalisation, le dispositif comprend également d'autres moyens permettant de réaliser des échanges avec un d'autres dispositifs de mesure d'activité, comme des moyens d'enregistrement de rythmes cardiaques ou respiratoires.

Dans au moins un mode de réalisation, le dispositif se présente sous la forme d'un dispositif autonome, pilotable à distance, par exemple par l'intermédiaire d'un terminal de type tablette, Smartphone ou ordinateur. Dans ce mode de réalisation le dispositif peut ne pas avoir de finalité médicale.

Dans un autre mode de réalisation, le dispositif est mis en oeuvre sous la forme d'un module, par exemple un module matériel, intégré à une console de polysomnographie, module dans lequel le flux audio/vidéo d'origine est traité en continu, de manière à corréler le ou les indice représentatifs du niveau de repos d'un individu à d'autres indices.

Dans un autre mode de réalisation, le dispositif de l'invention est piloté par la console de polysomnographie pour être mis en oeuvre en cas de détection d'une anomalie ou d'une valeur particulière d'un autre paramètre de mesure enregistré par la console, telle qu'une élévation du rythme cardiaque afin de vérifier si cette élévation est lié à un état de veille ou de repos de l'individu étudié.

**Revendications**

1. Procédé de calcul d'un indice d'activité d'un individu mis en oeuvre par l'intermédiaire d'un dispositif de calcul d'indice, procédé **caractérisé en ce qu'**il comprend :

 - une étape d'obtention (10), à partir d'un flux audio /vidéo d'origine *(FlvO),* d'au moins deux flux *(x1, x2, x3),* chacun comprenant une pluralité d'échantillons horodatés ladite étape d'obtention (10) comprenant :

  - une étape d'obtention (300) de données représentative d'un état des yeux, délivrant un flux (x3) comprenant des échantillons relatifs à l'ouverture des yeux dudit individu.

 - une étape de formatage (20) desdits au moins deux flux *(x1, x2, x3),* délivrant au moins deux flux formatés *(x1f, x2f, x3f)* ;
 - une étape de combinaison (30), desdits au moins deux flux formatés, délivrant un flux combiné *(xc)* ;
 - une étape de calcul (40), à partir dudit flux combiné *(xc)* d'au moins un indice représentatif d'un niveau d'activité d'un individu *(IrNA).*

2. Procédé de calcul, selon la revendication 1, **caractérisé en ce que** ladite étape de combinaison (30) met en oeuvre une fonction de fusion *(ff)* :

$$xc_j = ff(x_j) = \sum_{i=1}^{n} \propto_i x_{ij}$$

 Dans laquelle :

  - *n* représente le nombre de flux ;
  - *j* représente le quantième de l'échantillon ;
  - $x_{ij}$ est la valeur du flux normalisé $x_i$ au numéro d'échantillon *j;*
  - $\alpha_i$ est une valeur de pondération du flux $x_i$.

3. Procédé de calcul, selon la revendication 2, **caractérisé en ce que** ladite valeur $\alpha_i$ de pondération du flux $x_i$ varie d'un quantième d'échantillon à un autre.

4. Procédé de calcul, selon la revendication 2, **caractérisé en ce que** ladite valeur $\alpha_i$ de pondération du flux $x_i$ est paramétrée en fonction d'au moins un flux $x_j$, $j \neq i$.

5. Procédé de calcul, selon la revendication 1, **caractérisé en ce qu'**une étape d'obtention (10), à partir d'un flux audio /vidéo d'origine *(FlvO),* d'au moins deux flux *(x1, x2, x3),* comprend en outre au moins une des étapes suivantes :

  - une étape d'estimation de mouvement (100), délivrant un flux (x1) comprenant des échantillons relatifs à l'évaluation du mouvement entre des images successives dudit d'un flux audio /vidéo d'origine *(FlvO)* ;
  - une étape de collecte d'intensité sonore (200), délivrant un flux (x2) comprenant des échantillons relatifs à l'évaluation d'un volume sonore.

**6.** Dispositif de calcul d'un indice d'activité d'un individu, mis en oeuvre par l'intermédiaire d'un dispositif de calcul d'indice, dispositif **caractérisé en ce qu'**il comprend :

- des moyens d'obtention (10), à partir d'un flux audio /vidéo d'origine *(FlvO)*, d'au moins deux flux *(x1, x2, x3)*, chacun comprenant une pluralité d'échantillons horodatés ;
- des moyens de formatage (20) desdits au moins deux flux *(x1, x2, x3)*, configurés pour délivrer au moins deux flux formatés ;
- des moyens de combinaison (30), desdits au moins deux flux formatés, configurés pour délivrer un flux combiné *(xc)* ;
- des moyens de calcul (40), à partir dudit flux combiné *(xc)* d'au moins un indice représentatif d'un niveau de repos d'un individu.

**7.** Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour l'exécution du procédé selon l'une au moins des revendications 1 à 5, lorsqu'il est exécuté sur un ordinateur.

**8.** Procédé de calcul d'un indice de calme d'un individu mis en oeuvre par l'intermédiaire d'un dispositif de calcul d'indice, procédé **caractérisé en ce qu'**il comprend :

- une étape d'obtention (10), à partir d'un flux audio /vidéo d'origine *(FlvO)*, de deux flux *(x1, x2)*, chacun comprenant une pluralité d'échantillons horodatés ladite étape d'obtention (10) comprenant :

- une étape d'estimation de mouvement (100), délivrant un flux (x1) comprenant des échantillons relatifs à l'évaluation du mouvement entre des images successives dudit d'un flux audio /vidéo d'origine *(FlvO)* ;
- une étape de collecte d'intensité sonore (200), délivrant un flux (x2) comprenant des échantillons relatifs à l'évaluation d'un volume sonore.

- une étape de formatage (20) desdits deux flux *(x1, x2)*, délivrant au moins deux flux formatés *(x1f, x2f)* ;
- une étape de combinaison (30), desdits au moins deux flux formatés, délivrant un flux combiné *(xc)* ;
- une étape de calcul (40), à partir dudit flux combiné *(xc)* d'au moins un indice de calme d'un individu *(IrNA)*.

**Patentansprüche**

**1.** Verfahren zur Berechnung eines Aktivitätsindex eines Individuums, das mit Hilfe einer Vorrichtung zur Indexberechnung eingesetzt wird, **dadurch gekennzeichnet, dass** es umfasst:

- einen Schritt der Erfassung (10) mindestens zweier Ströme (x1, x2, x3) aus einem ursprünglichen Audio-/Videostrom (FlvO), wobei jeder eine Vielzahl von zeitgesteuerten Proben umfasst, wobei der Erfassungsschritt (10) umfasst:

- einen Schritt der Erfassung (300) von Daten, die für einen Zustand der Augen repräsentativ sind, der einen Strom (x3) liefert, der Proben im Hinblick auf die Öffnung der Augen des Individuums umfasst,

- einen Schritt der Formatierung (20) der mindestens zwei Ströme (x1, x2, x3), der mindestens zwei formatierte Ströme (x1f, x2f, x3f) liefert;
- einen Schritt der Kombination (30) der mindestens zwei formatierten Ströme, der einen kombinierten Strom (xc) liefert;
- einen Schritt der Berechnung (40) mindestens eines für ein Aktivitätsniveau eines Individuums repräsentativen Indexes (IrNA).

**2.** Verfahren zur Berechnung nach Anspruch 1, **dadurch gekennzeichnet, dass** der Schritt der Kombination (30) eine Fusionsfunktion (ff) einsetzt:

$$xc_j = ff(x_j) = \sum_{i=1}^{n} \propto_i x_{ij}$$

wobei:

- n die Anzahl von Strömen darstellt;
- j das Datum der Probe darstellt;
- $x_{ij}$ der Wert des normalisierten Stroms $x_i$ zur Probenummer j ist;
- $\alpha_i$ ein Gewichtungswert des Stroms $x_i$ ist.

3. Verfahren zur Berechnung nach Anspruch 2, **dadurch gekennzeichnet, dass** der Gewichtungswert $\alpha_i$ des Stroms $x_i$ um einem Probendatum zu anderen variiert.

4. Verfahren zur Berechnung nach Anspruch 2, **dadurch gekennzeichnet, dass** der Gewichtungswert $\alpha_i$ des Stroms $x_i$ in Abhängigkeit von mindestens einem Strom $x_j$, $j \neq i$ parametriert ist.

5. Verfahren zur Berechnung nach Anspruch 1, **dadurch gekennzeichnet, dass** ein Schritt (10) der Erfassung mindestens zweier Ströme (x1, x2, x2) aus einem ursprünglichen Audio-/Videostrom (FlvO) ferner mindestens einen der folgenden Schritte umfasst:

- einen Schritt der Schätzung einer Bewegung (100), der einen Strom (x1) liefert, umfassend Proben in Zusammenhang mit der Bewertung der Bewegung zwischen aufeinanderfolgenden Bildern eines ursprünglichen Audio-/Videostroms (FlvO);
- einen Schritt des Sammelns einer Lautstärke (200), der einen Strom (x2) liefert, umfassend Proben in Zusammenhang mit der Bewertung eines Tonvolumens.

6. Vorrichtung zur Berechnung eines Aktivitätsindex eines Individuums, die mit Hilfe einer Vorrichtung zur Indexberechnung eingesetzt wird, **dadurch gekennzeichnet, dass** sie umfasst:

- Mittel zur Erfassung (10) mindestens zweier Ströme (x1, x2, x2) aus einem ursprünglichen Audio-/Videostrom (FlvO), wobei jedes eine Vielzahl von zeitgesteuerten Proben umfasst;
- Mittel zur Formatierung (20) der mindestens zwei Ströme (x1, x2, x3), die dazu vorgesehen sind, mindestens zwei formatierte Ströme (x1f, x2f, x3f) zu liefern;
- Mittel zur Kombination (30) der mindestens zwei formatierten Ströme, die dazu vorgesehen sind, einen kombinierten Strom (xc) zu liefern;
- Mittel zur Berechnung (40) mindestens eines für ein Ruheniveau eines Individuums repräsentativen Indexes.

7. Computerprogrammprodukt, das von einem Kommunikationsnetz fernladbar und/oder auf einem von einem Computer lesbaren Träger speicherbar und/oder von einem Mikroprozessor ausführbar ist, **dadurch gekennzeichnet, dass** es Programmcodebefehle für die Ausführung des Verfahrens nach mindestens einem der Ansprüche 1 bis 5 umfasst, wenn es auf einem Computer ausgeführt wird.

8. Verfahren zur Berechnung eines Aktivitätsindex eines Individuums, das mit Hilfe einer Vorrichtung zur Indexberechnung eingesetzt wird, wobei das Verfahren **dadurch gekennzeichnet ist, dass** es umfasst:

- einen Schritt der Erfassung (10) zweier Ströme (x1, x2) aus einem ursprünglichen Audio-/Videostrom (FlvO), wobei jeder eine Vielzahl von zeitgesteuerten Proben umfasst, wobei der Erfassungsschritt (10) umfasst:

- einen Schritt der Schätzung einer Bewegung (100), der einen Strom (x1) liefert, umfassend Proben in Zusammenhang mit der Bewertung der Bewegung zwischen aufeinanderfolgenden Bildern eines ursprünglichen Audio-/Videostroms (FlvO);
- einen Schritt des Sammelns von Lautstärke (200), der einen Strom (x2) liefert, umfassen Proben in Zusammenhang mit der Bewertung eines Tonvolumens,

- einen Schritt der Formatierung (20) der zwei Ströme (x1, x2), der mindestens zwei formatierte Ströme (x1f, x2f) liefert;

- einen Schritt der Kombination (30) der mindestens zwei formatierten Ströme, der einen kombinierten Strom (xc) liefert;
- einen Schritt der Berechnung (40) mindestens eines Ruheindexes eines Individuums (IrNA).

**Claims**

1. Method of calculating an activity index of an individual implemented by the intermediary of an index calculation device, method **characterised in that** it comprises:

   - a step of obtaining (10), from an original audio/video flow *(FlvO)*, at least two flows *(x1, x2, x3),* each one comprising multiple time-stamped samples, said obtaining step (10) comprising:

      - a step of obtaining (300) data representative of an eye condition, delivering a flow (x3) comprising samples relating to the opening of the eyes of said individual,

   - a step of formatting (20) said at least two flows *(x1, x2, x3)*, delivering at least two formatted flows *(x1f, x2f, x3f)*;
   - a step of combining (30) said at least two formatted flows, delivering a combined flow *(xc)*;
   - a step of calculating (40), from said combined flow *(xc)*, at least one index representative of a level of activity of an individual *(IrNA)*.

2. Method of calculating, according to Claim 1, **characterised in that** said combination step (30) implements a fusion function *(ff)*;

$$xc_j = ff(x_j) = \sum_{i=1}^{n} \propto_i x_{ij}$$

Wherein:

   - *n* represents the number of flows;
   - *j* represents the sample number;
   - $x_{ij}$ is the value of the standardised flow $x_j$ to the sample number *j*;
   - $\alpha_i$ is a weighting value of the flow $x_i$;

3. Method of calculating, according to Claim 2, **characterised in that** said weighting value $\alpha_i$ of flow $x_i$ varies from one sample number to another.

4. Method of calculating, according to Claim 2, **characterised in that** said weighting value $\alpha_i$ of flow $x_i$ is configured according to at least one flow $x_j$, $j \neq i$.

5. Method of calculating, according to Claim 1, **characterised in that** a step of obtaining (10), from an original audio/video flow *(FlvO)*, at least two flows *(x1, x2, x3),* further comprises at least one of the following steps:

   - a step of estimating movement (100), delivering a flow (x1) comprising samples relating to the evaluation of the movement between successive images of said original audio/video flow *(FlvO)*;
   - a step of collecting sound intensity (200), delivering a flow (x2) comprising samples relating to the evaluation of a sound volume.

6. Device for calculating an activity index of an individual, implemented by the intermediary of an index-calculating device, device **characterised in that** it comprises:

   - means for obtaining (10), from an original audio/video flow *(FlvO)*, at least two flows *(x1, x2, x3),* each one comprising multiple time-stamped samples;
   - means for formatting (20) said at least two flows *(x1, x2, x3)*, configured to deliver at least two formatted flows;
   - means for combining (30), said at least two formatted flows, configured to deliver one combined flow *(xc)*;

- means for calculating (40), from said combined flow *(xc)* at least one index representative of a rest level of an individual.

7. Computer program product downloadable from a communication network and/or stored on media readable by a computer and/or executable by a microprocessor, **characterised in that** it comprises program code instruction to execute the method according to at least one of Claims 1 to 5, when it is executed by a computer.

8. Method for calculating a calm index of an individual implemented by the intermediary of an index calculation device, method **characterised in that** it comprises:

   - a step of obtaining (10), from an original audio/video flow *(FlvO)*, two flows *(x1, x2)*, each one comprising multiple time-stamped samples, said obtaining step (10) comprising:

      - a step of estimating movement (100), delivering a flow (x1) comprising samples relating to the evaluation of the movement between successive images of said original audio/video flow *(FlvO)*;
      - a step of collecting sound intensity (200), delivering a flow (x2) comprising samples relating to the evaluation of a sound volume,

   - a step of formatting (20) said at least two flows *(x1, x2, x3),* delivering at least two formatted flows *(x1f, x2f)*;
   - a step of combining (30) said at least two formatted flows, delivering a combined flow *(xc)*;
   - a step of calculating (40), from said combined flow *(xc),* at least one calm index of an individual *(IrNA)*.

Figure 1

Figure 2

Figure 3

**EP 3 073 900 B1**

## RÉFÉRENCES CITÉES DANS LA DESCRIPTION

**Documents brevets cités dans la description**

- WO 2013103700 A1
- US 5689241 A